Europäisches Patentamt

European Patent Office

Office européen des brevets

(11) Numéro de publication: **0 291 616 B1**

(12) **FASCICULE DE BREVET EUROPEEN**

(45) Date de publication de fascicule du brevet: **27.05.92**  (51) Int. Cl.5: **A61L 2/22**, A61L 9/14

(21) Numéro de dépôt: **87440027.8**

(22) Date de dépôt: **13.05.87**

(54) **Installation de désinfection des surfaces par voie aérienne dans des enceintes closes.**

(43) Date de publication de la demande:
**23.11.88 Bulletin 88/47**

(45) Mention de la délivrance du brevet:
**27.05.92 Bulletin 92/22**

(84) Etats contractants désignés:
**AT BE CH DE ES GB GR IT LI LU NL SE**

(56) Documents cités:
**WO-A-79/01074**
**FR-A- 2 000 622**
**FR-A- 2 366 842**
**FR-A- 2 414 337**
**FR-A- 2 448 930**

(73) Titulaire: **Laboratoires ANIOS S.A.**
**Rue Pavé du Moulin**
**F-59260 Lille-Hellemmes (Nord)(FR)**

(72) Inventeur: **Letartre, Thierry**
**6 Allée de la Cerisaie**
**59700 Marcq-en-Baroeuil(FR)**

(74) Mandataire: **Lepage, Jean-Pierre**
**Cabinet Lepage & Aubertin Innovations et**
**Prestations S.A. 23/25, rue Nicolas Leblanc**
**B.P. 1069**
**F-59011 Lille Cédex 1 (Nord)(FR)**

EP 0 291 616 B1

## Description

L'invention est relative à une installation de désinfection des surfaces par voie aérienne dans des enceintes closes. Elle trouvera notamment son application dans le milieu médical pour l'aseptie des salles de chirurgie.

Il est connu de procéder régulièrement à la décontamination des salles de chirurgie afin d'éviter le développement des micro-organismes.

Bien qu'un lavage doit être envisagé, il est préférable d'utiliser la voie aérienne pour procéder à la décontamination des surfaces. Selon ce procédé, on vaporise dans l'enceinte à désinfecter une solution chargée de matières actives possédant des propriétés désinfectantes. En laissant agir la solution durant une période appropriée, l'air chargé de la solution de désinfection balaie l'ensemble des parois de la pièce qui se voit ainsi désinfectée.

Des appareillages spécialisés ont été mis au point pour atteindre cet objectif. On peut les classer en deux catégories distinctes selon le mode de diffusion de la solution de désinfection utilisée.

Le type d'appareil le plus couramment rencontré présente des caractéristiques de construction qui assurent simultanément le brassage de l'atmosphère de la pièce dans laquelle il est placé, et la pulvérisation de la solution de désinfection.

Il se forme ainsi un brouillard dont les gouttes sont pulvérisées par une série de chicanes disposées sur la périphérie de l'hélice. Des micro-gouttelettes sont emportées par le brassage de l'air qui ainsi se saturent par contact avec la solution de désinfection.

Cet appareil présente l'avantage d'une grande simplicité mais n'est pas toujours très efficace dans la pulvérisation de la solution de désinfection. En particulier, généralement il se produit une sursaturation en solution de désinfection de l'air brassé et il y a condensation à proximité de l'appareil.

Si le brouillard n'est pas suffisamment formé de gouttelettes finement pulvérisées, les gouttes sont emportées par le courant d'air en quantités trop importantes pour être vaporisées d'où naissance d'une imprégnation humide des surfaces à proximité de l'appareil. Ce phénomène est particulièrement néfaste car il en découle une désinfection non homogène du local et une surconsommation de solution de désinfection.

Pour pallier cet inconvénient, il a été développé une seconde catégorie d'appareils dans laquelle il est possible de régler le débit d'éjection de la solution de désinfection. Ces appareils sont composés d'un dispositif de brassage de l'atmosphère du local dans lequel ils sont placés et d'un système indépendant de pulvérisation d'une solution de désinfection.

La pulvérisation est effectuée au niveau de la veine de brassage de sorte à vaporiser dans l'ensemble de l'air brassé la solution de désinfection. Pour atteindre cet objectif, on pulvérise à l'aide d'un gicleur et d'une pompe la solution en un fin brouillard emporté par un courant d'air.

Malheureusement, ce type d'appareil présente un rendement de fonctionnement dépendant des conditions climatiques rencontrées dans le local. En effet, pour éviter le phénomène de sursaturation rencontré avec la première catégorie d'appareil, il est nécessaire de régler le débit d'éjection de la solution pulvérisée de telle sorte à ne pas dépasser le seuil de saturation de l'atmosphère.

Cela étant, on connaît, notamment du document FR-A-2.366.842 ou WO-A-7.901.074 des installations pour la stérilisation d'objets, tels que des récipients d'emballages pour produits alimentaires. Ces installations comprennent d'une part des moyens pour pulvériser en un brouillard un agent désinfectant, puis éventuellement le vaporiser, et d'autre part des moyens pour véhiculer le dit agent désinfectant vers la ou les surfaces à désinfecter.

Il est à noter toutefois que ces dispositifs sont prévus pour la stérilisation de volumes très petits de l'ordre de quelques centimètres cube. Ainsi, de telles installations, notamment de par leur débit, sont totalement inadaptées à la désinfection d'un local d'un volume important tel que par exemple des salles de chirurgie.

Par ailleurs, si l'on cherchait à utiliser ces dispositifs pour un tel usage, on serait alors amené à augmenter le débit de la solution de désinfection et on retomberait dans les problèmes et inconvénients posés par les installations connues dont les inconvénients ont été repris ci-dessus.

Le but principal de la présente invention est de présenter une installation de désinfection des surfaces par voie aérienne dans les enceintes closes qui permette d'éviter tous les phénomènes de condensation de la solution de désinfection pulvérisée et qui en outre présente un rendement de vaporisation élevé.

La non-condensation de la solution de désinfection permet d'éviter tous les inconvénients précédemment rencontrés qui se traduisent par une humidification locale des parois situées à proximité de l'appareil, et par ailleurs, permet d'économiser la solution de désinfection en la limitant aux stricts besoins.

En outre, l'installation de la présente invention s'affranchit des conditions climatiques rencontrées et présente un pouvoir de vaporisation renforcé afin qu'elle puisse fonctionner même dans une atmosphère fortement saturée.

Un autre but de la présente invention est de présenter une installation de désinfection dont la mise en oeuvre présente une grande simplicité.

L'appareillage proposé est mobile et autonome ce qui autorisera son emploi dans différents locaux. Par ailleurs, les réglages sont simples et se limitent à une estimation du volume de la pièce à désinfecter.

D'autres buts et avantages de la présente invention apparaîtront au cours de la description qui va suivre, qui n'est cependant donnée qu'à titre indicatif et qui n'a pas pour but de la limiter.

Selon l'invention, l'installation de désinfection par voie aérienne d'un local, qui trouvera son application pour la désinfection des surfaces d'enceintes closes, tel que notamment dans le domaine médical pour l'aseptie des salles de chirurgie, la dite installation étant destinée à vaporiser dans l'atmosphère du dit local à désinfecter un agent désinfectant tel qu'une quantité dosée de solution de désinfection, et comprenant :

- des moyens pour pulvériser en un brouillard l'agent désinfectant, puis pour le vaporiser, et enfin pour véhiculer l'agent désinfectant vers la ou les surfaces à désinfecter,

est caractérisée par le fait que les dits moyens sont constitués par :

- un conduit assujetti à des moyens de brassage aptes à engendrer une veine d'air et à assurer une circulation de l'air présent dans le dit local à désinfecter au-travers de la dite installation,
- une buse d'éjection à gicleur placé au niveau du dit conduit dans la veine d'air en circulation, et recevant la dite quantité dosée de solution de désinfection par l'intermédiaire d'une pompe,
- une chambre annulaire pressurisée, prévue au niveau de la dite buse d'éjection, pour délivrer autour du dit gicleur un courant vecteur qui favorise la diffusion de la solution pulvérisée au sein de la veine d'air et crée une atmosphère non saturée dans la zone d'éjection du gicleur.

L'invention sera mieux comprise à la lecture de la description suivante accompagnée de dessins en annexe parmi lesquels :

- la figure 1 schématise les différents organes fonctionnels composant l'installation de désinfection de la présente invention,
- la figure 2 schématise un mode préférentiel de réalisation de l'installation de désinfection de la présente invention,
- la figure 3 illustre en vue de coupe un mode préférentiel de réalisation de la buse d'éjection de l'installation de la présente invention.

La présente invention vise une installation de désinfection des surfaces par voie aérienne dans des enceintes closes. Elle trouvera notamment son application dans le milieu médical pour l'aseptie des salles de chirurgie.

On connaît de longue date le procédé de désinfection des locaux médicaux en vaporisant dans l'atmosphère des dits locaux une solution de désinfection.

Toute la difficulté réside dans la vaporisation de la solution de désinfection. En effet, pour des raisons économiques et pratiques, il est nécessaire de réaliser cette opération dans un temps aussi court que possible. Toutefois, si l'on tente de vaporiser une quantité de solution de désinfection supérieure au pouvoir d'absorption de l'atmosphère, il s'en suit une condensation de la solution qui humidifie les parois du local situées à proximité de l'appareillage. Cette condensation est nuisible, elle représente une salissure et en outre un gaspillage de produit.

Le but recherché consiste donc à atteindre aussi près que possible le seuil de saturation de l'atmosphère sans jamais le dépasser. Toutefois, ce seuil de saturation est variable, ce qui rend l'opération délicate.

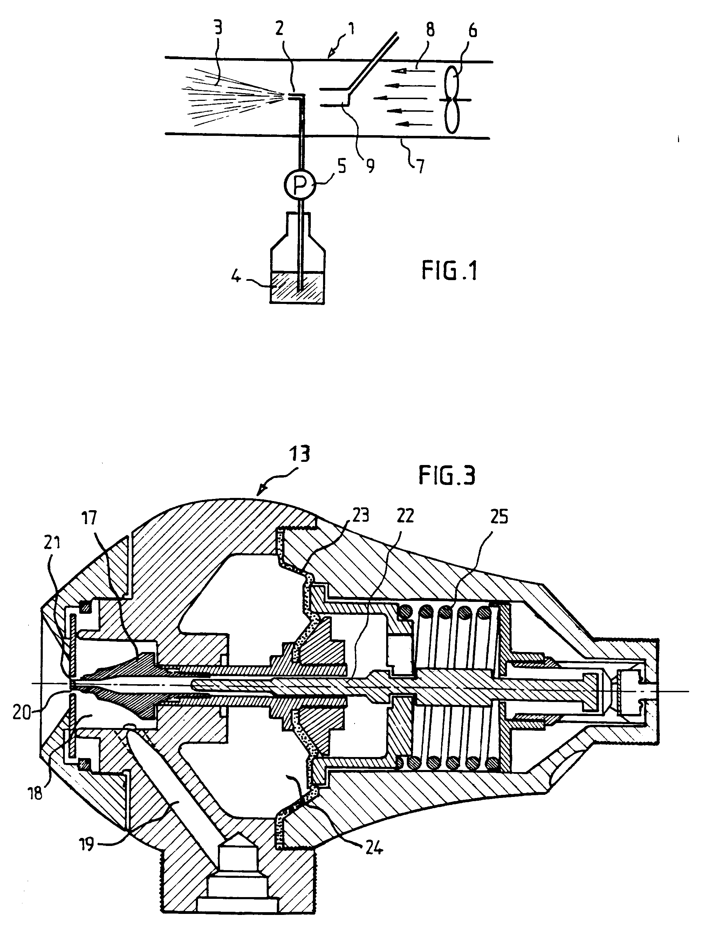

Cet objectif est atteint avec l'installation de désinfection de la présente invention schématisé à la figure 1.

L'installation de désinfection 1 se compose tout d'abord d'un dispositif de pulvérisation 2 en un brouillard 3 de la solution de désinfection 4. La pulvérisation en fines gouttelettes augmente la surface d'échange entre liquide et gaz et favorise la vaporation.

Ce dispositif de vaporisation peut par exemple se présenter sous la forme d'une buse 2 dans laquelle on introduit la solution de désinfection 4 sous pression par l'intermédiaire d'une pompe 5.

La pompe 5 permet en outre de contrôler le débit et procure une certaine force d'éjection à la pulvérisation.

L'installation 1 comprend en outre des moyens de brassage 6 qui assurent la circulation de l'air présent dans l'enceinte close à travers l'installation 1.

La pulvérisation de la solution de désinfection en un brouillard 3 est opérée dans le circuit de brassage de l'air de sorte à répandre dans le volume de l'enceinte à désinfecter la solution vaporisée.

A cet égard, on pourra utiliser un conduit 7 dans lequel on fera circuler l'air 8 brassé par les moyens 6. La pulvérisation 3 sera également réalisée dans le conduit 7.

Selon la caractéristique principale de la présente invention, l'installation 1 comprend en outre des moyens de conditionnement 9 qui assurent un support de diffusion et créent une atmosphère non saturée dans la zone d'éjection du dispositif de pulvérisation.

Ces moyens 9 de conditionnement sont très importants car ils accroissent considérablement la vitesse de vaporisation de la solution de désinfection et évitent tout phénomène de condensation.

Les moyens de conditionnement 9 assurent tout d'abord un support de diffusion, c'est-à-dire qu'ils créent un courant vecteur au niveau de la pulvérisation 3 qui favorise la diffusion de la solution pulvérisée au sein du courant d'air 8.

La pulvérisation à l'aide d'une buse á gicleur 2 est un phénomène très localisé et par conséquent il en découle une saturation locale très rapide qui nuit à la vaporisation totale de la solution. En utilisant un courant vecteur, par exemple produit par un souffle d'air rapide à ce niveau, il est possible de propulser les fines gouttelettes pulvérisées suffisamment loin de la zone de saturation locale pour que celles-ci puissent venir en contact avec de l'air non saturé qui absorbera les gouttelettes vaporisées.

En outre, les moyens de conditionnement 9 créent une atmosphère non saturée dans la zone de gestion du dispositif de pulvérisation 2. Cela signifie que quelles que soient les conditions atmosphériques extérieures, la pulvérisation en un brouillard 3 de la solution de désinfection 4 se fait dans une atmosphère qui a un pouvoir absorbant encore important. De la sorte, il est possible de procéder à la désinfection d'une pièce qui pourtant présente une atmosphère fortement saturée en humidité.

Pour créer ces conditions favorables à la pulvérisation, plusieurs moyens de conditionnement 9 peuvent être utilisés. Toutefois, le mode préférentiel retenu pour la présente invention est un groupe compresseur dont la production d'air comprimé est libérée au niveau de la pulvérisation 3 de la solution de désinfection 4.

Cette propriété est mise à profit dans la présente invention qui utilise de l'air préalablement comprimé pour procéder à la diffusion de la vaporisation 3 dans le courant d'air 8.

Les conditions de vaporisation sont ainsi rendues nettement moins dépendantes des conditions atmosphériques rencontrées dans le local à désinfecter.

Dans ces conditions, le débit de solution de désinfection 4 pulvérisé n'a pas à être ajusté à chaque cas d'espèces rencontrés. Ce débit est rendu constant et peut être dosé grâce à l'utilisation d'une pompe péristaltique 5.

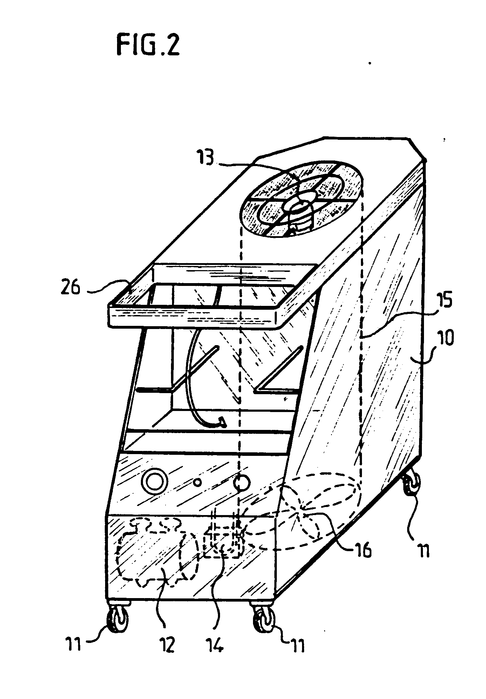

Sur le plan de la réalisation, la figure 2 illustre une conception préférentielle de l'installation de désinfection de la présente invention. Celle-ci se présente sous la forme d'un chariot 10 monté sur roulettes 11 et munie d'une poignée 26 de transport.

Ce chariot caréné 10 comprend un groupe compresseur 12 de préférence du type à membrane pour éviter toute déperdition d'huile dans le circuit. Ce groupe compresseur permet de créer des conditions de pulvérisation au niveau de l'élément de pulvérisation et de vaporisation 13 favorable à la vaporisation de la solution de désinfection qui alimente le dit élément 13 par l'intermédiaire d'une pompe péristaltique 14.

Le chariot 10 est en outre traversé par une gaine verticale 15 à l'intérieur de laquelle est placé un ventilateur 16 qui assure le brassage de l'atmosphère dans laquelle est placé le chariot 10.

Il faut souligner que la disposition verticale du conduit 15 est souhaitable. En effet, dans ce cas, les forces de gravitation qui agissent sur les gouttelettes pulvérisées de solution de désinfection n'ont pas tendance à déporter celles-ci en dehors du flux d'air brassé par le ventilateur 16.

La figure 3 schématise un mode préférentiel de réalisation de l'élément de pulvérisation et de vaporisation 13. Celui-ci comprend une buse d'éjection à gicleur 17 par l'intermédiaire de laquelle la solution est pulvérisée. Ce gicleur 17 est entouré par une chambre annulaire 18 pressurisée grâce à un canal 19 d'amenée d'air comprimé. La chambre annulaire 18 communique avec l'atmosphère par l'intermédiaire d'un espace annulaire 20 disposé autour de l'orifice 21 du gicleur 17. Ainsi, la pulvérisation de la solution est réalisée dans un courant d'air déjà animé d'une certaine vitesse ce qui favorise la diffusion du produit.

Un pointeau 22 qui agit sur l'orifice 21 du gicleur 17 est manoeuvré par l'intermédiaire d'une membrane 23 placée dans une chambre 24 dans laquelle est introduit de l'air comprimé de sorte à dégager le pointeau de l'orifice 21. En cas d'absence d'air comprimé, un ressort 25 agit sur le pointeau de sorte à obturer l'orifice 21 du gicleur 17.

## Revendications

1. Installation de désinfection par voie aérienne d'un local, qui trouvera son application pour la désinfection des surfaces d'enceintes closes, tel que notamment dans le domaine médical pour l'aseptie des salles de chirurgie, la dite installation étant destinée à vaporiser dans l'atmosphère du dit local à désinfecter un agent désinfectant tel qu'une quantité dosée de solution de désinfection, et comprenant :

   - des moyens (2, 9, 6, 7; 17, 18, 16, 15) pour pulvériser en un brouillard l'agent désinfectant, puis pour le vaporiser, et enfin pour véhiculer l'agent désinfectant vers la ou les surfaces à désinfecter, caractérisée par le fait que les dits moyens (2, 9, 6, 7; 17,18, 16,15) sont constitués par :

   - un conduit (7 ; 15) assujetti à des moyens (6; 16) de brassage aptes à engendrer une veine d'air (8) et à assurer une circulation de l'air présent dans le dit local à désinfecter au-travers de la dite installation (1),

- une buse d'éjection, munie d'un gicleur (2 ; 17) placé au niveau du dit conduit (7 ; 15) dans la veine (8) d'air en circulation, et recevant la dite quantité dosée de solution de désinfection (4) par l'intermédiaire d'une pompe (5; 14),
- une chambre annulaire (9 ; 18) pressurisée, prévue au niveau de la dite buse d'éjection, pour délivrer autour du dit gicleur (2 ; 17) un courant vecteur qui favorise la diffusion de la solution pulvérisée au sein de la veine d'air (8) et crée une atmosphère non saturée dans la zone d'éjection du gicleur.

2. Installation de désinfection selon la revendication 1, caractérisée par le fait que les moyens de brassage (6) se présentent sous la forme d'un ventilateur (16) placé dans un conduit (15) débouchant à ses extrémités dans l'enceinte dans laquelle est placé le dispositif de désinfection (1).

3. Installation de désinfection selon la revendication 1, caractérisée par le fait que le dosage de la solution de désinfection (4) est assuré au moyen d'une pompe (5) péristaltique qui alimente la buse á gicleur (2).

4. Installation de désinfection selon la revendication 1, caractérisée par le fait que la buse (2) est formée d'un gicleur (17) entouré d'une chambre annulaire (18) pressurisée communiquant avec l'atmosphère par un espace annulaire (20) disposé autour de l'orifice (21) du gicleur (17).

5. Installation de désinfection selon la revendication 4, caractérisée par le fait qu'elle présente un groupe compresseur (12) dont la production d'air comprimé est libérée dans la chambre annulaire (18) au niveau de la pulvérisation (3) de la solution de désinfection (4).

6. Installation de désinfection selon la revendication 4, caractérisée par le fait qu'elle comporte des moyens pour obturer l'orifice (21) du gicleur (17) commandé par la pressurisation de la chambre (18).

7. Installation de désinfection selon la revendication 6, caractérisée par le fait qu'un pointeau (22) manoeuvré par l'air comprimé de la chambre annulaire (18) commande l'ouverture de l'orifice (21) du gicleur (17).

8. Installation de désinfection selon la revendication 1, caractérisée par le fait que le conduit dans lequel sont placés les moyens de brassage est disposé verticalement.

**Claims**

1. Installation for aerial disinfection of premises, which will find an application in the disinfection of the surfaces of enclosed areas such as, in particular, in the medical field, for ensuring the asepsis of the operating rooms, the said installation being designed to spray in the atmosphere of the said premises for disinfection a disinfecting agent such as a proportioned quantity of disinfecting solution, and including:
   - means (2, 9, 6, 7; 17, 18, 16, 15) for pulverizing the disinfecting agent to form a mist, and then vaporizing it and, finally, for conveying the disinfecting agent to the surface or surfaces to be disinfected, characterized by the fact that the said means (2, 9, 6, 7; 17, 18, 16, 15) are formed by:
   - a conduit (7; 15) secured to stirring means (6; 16) suitable for generating a stream of air (8) and for ensuring circulation of the air present on the said premises to be disinfected through the said installation (1),
   - an discharge nozzle fitted with a jet (2, 17) placed in the area of the said conduit (7, 15) in the circulating stream of air (8), and receiving the said proportioned quantity of disinfecting solution (4) via a pump (5; 14),
   - a pressurized annular chamber (9; 18) provided in the area of the said discharge nozzle to supply around the said jet (2; 17) a vector current which promotes the diffusion of the pulverized solution within the stream of air (81 and creates a non-saturated atmosphere in the discharge zone of the jet,

2. Disinfecting installation according to claim 1, characterized by the fact that the stirring means (6) take the form of a fan (16) placed in a conduit (15) the ends of which emerge in the enclosed area in which the disinfecting device (1) is placed.

3. Disinfecting installation according to claim 1, characterized by the fact that the disinfecting solution (4) is proportioned by means of a peristaltic pump (5) which supplies the nozzled fitted with the jet (2),

4. Disinfecting installation according to claim 1, characterized by the fact that the nozzle (2) is formed by a jet (17) surrounded by a pressurized annular chamber (18) commnicating with atmosphere via an annular space (20) provided around the orifice (21) of the jet (17),

5. Disinfecting installation according to claim 4, characterized by the fact that it has a compressor unit (12), the compressed air from which is released into the annular chamber (18) in the area of the pulverization (3) of the disinfecting solution (4).

6. Disinfecting installation according to claim 4, characterized by the fact that it includes means for closing the orifice (21) of the jet (17) controlled by the pressurization of the chamber (18).

7. Disinfecting installation according to claim 6, characterized by the fact that a needle valve (22) actuated by the compressed air in the annular chamber (18) controls the opening of the orifice (21) of the jet (17).

8. Disinfecting installation according to claim 1, characterized by the fact that the conduit in which the stirring means are placed is disposed vertically.

**Patentansprüche**

1. Anlage zur Entseuchung eines Raums über die Luft, die ihre Anwendung zur Entseuchung der Oberflächen geschlossener Räume finden wird, wie nämlich auf dem medizinischen Gebiet zur Asepsis der Operationssäle, wobei die genannte Anlage dazu bestimmt ist, ein Entseuchungsmittel, wie eine dosierte Menge Entseuchungslösung, in die Luft des zu entseuchenden Raums zu verdunsten, umfassend:
   - Mittel (2, 9, 6, 7; 17, 18, 16, 15) zum Zerstäuben in Nebel, danach zum Verdunsten des Entseuchungsmittels und schließlich zum Befördern des Entseuchungsmittels zu der oder den zu entseuchenden Oberfläche(n),
   dadurch gekennzeichnet, daß die genannten Mittel (2, 9, 6, 7; 17, 18, 16, 15) bestehen aus:
   - einer Leitung (17; 15), die Umrührmitteln (6; 16) unterworfen ist, die geeignet sind, eine Luftader (8) zu erzeugen und einen Umlauf der im genannten zu entseuchenden Raum anwesenden Luft durch die gennante Anlage (1) zu sichern,
   - einem mit einer Sprühdüse (2; 17) versehenen Auswerfrohr, das im Bereich der genannten Leitung (7; 15) in der umlaufenden Luftader (8) angeordnet ist und die genannte dosierte Menge Entseuchungsmittel (4) über eine Rumpe (5; 14) erhält,
   - einer im Bereich des genannten Auswerfrohrs vorgesehenen, unter Druck stehenden ringförmigen Kammer (9; 18) zur Versorgung um um die genannte Sprühdüse (2; 17) herum eines Vektorstroms, der die Verbreitung der zerstäubten Lösung innerhalb der Luftader (8) befördert und zu einer nichtgesättigten Luft im Auswurfbereich der Sprühdüse führt.

2. Entseuchungsanlage nach Anspruch 1, dadurch gekennzeichnet, daß die Umrührmittel (6) als ein in einer Leitung (15), die an deren Enden in den Raum mündet, in dem die Entseuchungsvorrichtung (1) angeordnet ist, angeordneter Ventilator (16) ausgestaltet sind.

3. Entseuchungsanlage nach Anspruch 1, dadurch gekennzeichnet, daß die Dosierung der Entseuchungslösung (4) mittels einer Peristaltikpumpe (5), die das Sprühdüsenrohr (2) speist, gesichert wird.

4. Entseuchungsanlage nach Anspruch 1, dadurch gekennzeichnet, daß das Rohr (2) aus einer Sprühdüse (17) gebildet ist, die von einer unter Druck stehenden ringförmigen Kammer (18) umschlossen ist, die über einen um die Öffnung (21) der Sprühdüse (17) herum angeordneten ringförmigen Hohlraum (20) mit der Luft verbunden ist.

5. Entseuchungsanlage nach Anspruch 4, dadurch gekennzeichnet, daß sie ein Verdichteraggregat (12) aufweist, dessen Preßluftproduktion im Bereich der Zerstäubung (3) der Entseuchungslösung (4) in der ringförmigen Kammer (18) freigegeben wird.

6. Entseuchungsanlage nach Anspruch 4, dadurch gekennzeichnet, daß sie von der Unterdrucksetzung der Kammer (18) gesteuerte Mittel zum Verschließen der Öffnung (21) der Sprühdüse (17) umfaßt.

7. Entseuchungsanlage nach Anspruch 6, dadurch gekennzeichnet, daß eine von der Preßluft der ringförmigen Kammer (18) betätigte Nadel (22) das Öffnen der Öffnung (21) der Sprühdüse (17) steuert.

8. Entseuchungsanlage nach Anspruch 1, dadurch gekennzeichnet, daß die Leitung, in der die Umrührmittel angeordnet sind, senkrecht angeordnet ist.

FIG.1

FIG.3

FIG.2